# EUROPEAN PATENT APPLICATION

(11) **EP 4 717 263 A2**
(43) Date of publication of application: **01.04.2026**
(21) Application number: 26156991.7
(22) Date of filing: 10.01.2023
(51) Int. Cl.: A61K 9/48

(54) **DRUG DELIVERY VEHICLES, METHODS OF PRODUCING DRUG DELIVERY VEHICLES, AND METHODS OF USING DRUG DELIVERY VEHICLES**

(30) Priority: 13.01.2022 US 202263266752 P
(62) Divisional of application: 23704037.3
(71) Applicant: Verily Life Sciences LLC, Dallas, TX 75019 (US)
(72) Inventor: LEE, Kye, San Francisco, 94080 (US); AHMAD, Habibullah, San Francisco, 94080 (US); LU, Bo, San Francisco, 94080 (US); PAOLI, Eric, San Francisco, 94080 (US); KAM, Kimberly, San Francisco, 94080 (US)
(74) Representative: Mewburn Ellis LLP

(57) **Abstract**

The present disclosure is related to a method of producing a capsule. The method includes providing a mold, depositing parylene on the mold to form a parylene layer, scoring the parylene layer, and coating the parylene layer with a polymer to produce a capsule.

## Description

### TECHNICAL FIELD

The present disclosure generally relates to drug delivery vehicles, methods for producing drug delivery vehicles, and methods of using drug delivery vehicles to deliver drugs or other payloads inside the body of a subject. More specifically, the present disclosure relates to ingestible drug delivery vehicles (e.g., capsules) to deliver a payload to a subject (e.g., to a lining of a gastrointestinal tract of the subject).

### BACKGROUND OF THE INVENTION

Drug delivery vehicles (e.g., capsules) are designed to deliver a payload (e.g., a pharmaceutical active ingredient (API) or mechanical actuator) to a desired region of the body. During delivery, the payload needs to remain stable to ensure efficacy and patient safety. The degradation of the payload can occur through thermal degradation, oxidation, UV, microorganisms, or hydrolysis. For example, the physical and chemical properties of pharmaceutical solids may be affected by moisture.

Moisture can affect stability due to its influence on the glass transition temperature of pharmaceutical active ingredients. Many APIs and excipients are hygroscopic in nature and need to be protected from moisture. Specifically, moisture may have a significant impact on a wide range of chemical, physical, and microbial properties of the finished pharmaceutical product, leading to a degradation of the API and a subsequent loss of potency. Therefore, drug delivery vehicles including such ingredients need to be designed to prevent degradation because moisture affects the performance of some drug products.

Despite the low moisture content present in conventional hydroxypropyl methylcellulose capsules, there are still some APIs that are highly hygroscopic and sensitive to moisture. Such hygroscopic and moisture-sensitive compounds could be challenging for formulators and may involve extra investment due to the need to apply different pathways to protect the API - i.e., galenic forms such as pellets or capsule-in-capsule technology, a final additional drying process after filling or the use of moisture-protective packaging materials. The possibility of drying the filled capsule to reduce the water content as much as possible exposes the whole filling to harsh drying conditions. Furthermore, adding an additional step in the process after the filling involves extra resources in terms of capabilities (with dedicated equipment, room, human resources and conditions) and timing, extending the process and thus decreasing the overall yield.

Thus, a need exists for improved drug delivery vehicles that prevent moisture and vapor ingress.

### SUMMARY OF THE INVENTION

The present disclosure relates to capsules, enteric coatings for capsules, methods of producing capsules, and methods of coating capsules. The capsules described herein prevent moisture and vapor ingress into the capsule during delivery of the payload. For example, the capsule may include a moisture-barrier layer that prevents moisture from entering interior volume of the capsule until delivery in the gastrointestinal tract. The capsules described herein also include an enteric coating that leaves little or no residue when dissolved. The friability of the enteric coating of the capsule advantageously allows for mechanical actuators to effectively deploy from the interior volume of the capsule.

Embodiments of the present disclosure include a method of producing a capsule. The method includes providing a mold, depositing a parylene on the mold to form a parylene layer, scoring the parylene layer, and coating the parylene layer with a polymer to produce a capsule. In some embodiments, the method includes polishing a surface of the mold to ease release of the capsule. In some embodiments, the method includes applying a release agent on the mold before deposition of the parylene layer. In some embodiments, the release agent is Micro90. In some embodiments, scoring the parylene layer comprises cutting the parylene to form a pattern on opposing sides of the capsule. In some embodiments, the method includes treating an outer surface of the parylene layer with oxygen plasma prior to coating with the polymer. In some embodiments, a thickness of the parylene layer ranges from 0.5 µm to 30 µm. In some embodiments, a thickness of the parylene layer ranges from 5 µm to 10 µm. In some embodiments, depositing the parylene layer comprises chemical vapor deposition of parylene on the mold. In some embodiments, depositing the parylene layer comprises depositing, in a first deposition step, parylene on the mold to produce a primer layer, treating the primer layer with a release agent, and depositing, in a second deposition step, parylene on the primer layer to produce the parylene layer. In some embodiments, wherein the polymer is polyvinyl alcohol. In some embodiments, coating the parylene layer with the polymer comprises a plurality of coating steps. In some embodiments, a thickness of the polymer layer ranges from 100 µm to 250 µm. In some embodiments, the method further comprises applying an enteric coating on the polymer layer. In some embodiments, the enteric coating comprises anionic methacrylate copolymer, talc, and plasticizer. In some embodiments, the method further comprises encapsulating a needle delivery system in the capsule.

Embodiments of the present disclosure include a capsule. The capsule includes a first layer comprising parylene. The capsule includes a second layer adjacent the first layer. The second layer comprises a polymer. The coating includes a third layer adjacent the second layer. The third layer comprises an enteric coating. In some embodiments, wherein the first layer is scored. In some embodiments, the first layer is scored to form a bi-fold pattern on opposing sides of the capsule. In some embodiments, a thickness of the parylene layer ranges from 0.5 µm to 30 µm. In some embodiments, the second layer comprises a plurality of layers, wherein at least one of the plurality of layers comprises polyvinyl alcohol. In some embodiments, the polymer comprises one or more of hydroxypropyl methylcellulose, pullulan, polyvinyl alcohol, or polyvinylpyrrolidone. In some embodiments, the second layer has a thickness from 100 µm to 250 µm. In some embodiments, the enteric coating comprises anionic methacrylate copolymer, talc, and plasticizer. In some embodiments, talc comprises 40 wt. % to 80 wt. % of the enteric coating, based on the total weight of the enteric coating. In some embodiments, anionic methacrylate copolymer comprises 20 wt. % to 60 wt. % of the enteric coating, based on the total weight of the enteric coating. In some embodiments, the first layer is surface treated with oxygen plasma. In some embodiments, the capsule does not comprise pullulan or hydroxypropyl methylcellulose. In some embodiments, the capsule comprises a fourth layer comprising anionic methacrylate copolymer and plasticizer. In some embodiments, the polymer of the second layer comprises polyvinyl alcohol, the enteric coating comprises anionic methacrylate copolymer, talc, and plasticizer, and a fourth layer comprises anionic methacrylate copolymer and plasticizer.

Embodiments of the present disclosure include an enteric coating composition. The enteric coating composition may comprise an anionic methacrylate copolymer, talc, and plasticizer. In some embodiments, the enteric coating composition comprises 20 wt. % to 60 wt. % anionic methacrylate copolymer, based on the total weight of the composition. In some embodiments, the enteric coating composition comprises 40 wt. % to 80 wt. % of talc, based on the total weight of the composition. In some embodiments, a weight ratio of talc to anionic methacrylate copolymer ranges from 0.5:1 to 10:1. In some embodiments, a weight ratio of talc to anionic methacrylate copolymer ranges from 1:1 to 3:1. In some embodiments, the plasticizer is present in an amount from 5 wt. % to 25 wt. %, based on the total weight of the anionic methacrylate copolymer. In some embodiments, the plasticizer is hydrophobic. In some embodiments, the plasticizer comprises dibutyl sebacate. In some embodiments, a capsule comprising the enteric coating composition described herein.

Embodiments of the present disclosure include a method of producing a coating composition. The method includes providing a solvent comprising a plasticizer, adding anionic methacrylate copolymer to the solvent, adding talc to the solvent, and mixing the anionic methacrylate copolymer and talc in the solvent to produce the coating composition. In some embodiments, the plasticizer is hydrophobic. In some embodiments, the plasticizer comprises dibutyl sebacate. In some embodiments, the solvent comprises one or more of acetone, isopropyl alcohol, and water. In some embodiments, the plasticizer is present in an amount from 5 wt. % to 25 wt. %, based on the weight of the anionic methacrylate copolymer. In some embodiments, a weight ratio of talc to anionic methacrylate copolymer ranges from 0.5:1 to 10:1. In some embodiments, a weight ratio of talc to anionic methacrylate copolymer ranges from 1:1 to 3:1. In some embodiments, providing the solvent comprises mixing the plasticizer in the solvent prior to adding the enteric composition.

Embodiments of the present disclosure include a method of coating a capsule. The method includes providing an enteric solution comprising a solvent, a plasticizer, anionic methacrylate copolymer, and talc, coating a capsule with the enteric solution, and drying the enteric solution on the capsule to produce an enteric coating. In some embodiments, coating the capsule comprises dip coating the capsule in the enteric solution. In some embodiments, the enteric coating comprises 20 wt. % to 60 wt. % of anionic methacrylate copolymer, based on the total weight of the enteric coating. In some embodiments, the enteric coating comprises 40 wt. % to 80 wt. % of talc, based on the total weight of the enteric coating. In some embodiments, the method includes overcoating the capsule with a second enteric solution comprising anionic methacrylate copolymer and plasticizer. In some embodiments, the method includes coating one or more additional polymer layers on the capsule. In some embodiments, drying the enteric solution on the capsule comprises a first drying step comprising rotating the capsule in a rotisserie at ambient conditions, a second drying step comprising rotating the capsule in a rotisserie at ambient conditions with forced air, and a third drying step comprising drying the enteric solution in an oven.

Further aspects, objects, and advantages will become apparent upon consideration of the detailed description.

### BRIEF DESCRIPTION OF THE DRAWINGS

FIG. 1 provides a flow diagram of a method of producing a capsule according to some embodiments of the present disclosure.
FIG. 2A illustrates a capsule according to some embodiments of the present disclosure.
FIG. 2B illustrates a capsule including a needle delivery system according to some embodiments of the present disclosure.
FIGS. 3A and 3B illustrate an embodiment of a scored parylene layer of the capsule according to some embodiments of the present disclosure.
FIG. 4 illustrates a scoring pattern on the parylene layer of the capsule according to some embodiments of the present disclosure.
FIG. 5 provides a graph of the polymer thickness of the capsule according to some embodiments of the present disclosure.
FIGS. 6A-6C illustrate deploying a microneedle system from a capsule according to some embodiments of the present disclosure.
FIG. 7 provides a flow diagram of a method of coating a capsule with an enteric solution according to some embodiments of the present disclosure.
FIGS. 8A-8D illustrate a manufacturing process for producing a capsule according to some embodiments of the present disclosure.
FIG. 9 provides a qualitative graph of the dissolution rates and gastric protection for various HPMC capsules.
FIG. 10A-H provide images showing the residue of the capsule after dissolution according to some embodiments of the present disclosure.
FIG. 11A-F provide visibility tests showing moisture ingress in the capsules in simulated gastric fluid according to some embodiments of the present disclosure.
FIGS. 12A-12F show a process of producing capsules by melting and reflowing a polymer according to some embodiments of the present disclosure.
FIG. 13 provides a graph of the coating thickness for capsules produced from Kollicoat^{®} IR and polyvinyl alcohol (PVA) according to some embodiments of the present disclosure.
FIGS. 14A and 14B show images of capsules produced from Kollicoat^{®} IR deposited on a parylene layer (FIG. 14A) and capsules produced from Kollicoat^{®} IR without a parylene layer (FIG. 14B) according to some embodiments of the present disclosure.
FIGS. 15A-15D shows images of capsules including a Kollicoat^{®} IR layer and a parylene layer before (FIGS. 15A and 15C) and after (FIGS. 15B and 15D) in-vivo dissolution tests according to some embodiments of the present disclosure.
FIGS. 16A-16D shows images of capsules including a pullulan layer before (FIGS. 16A and 16C) and after (FIGS. 16B and 16D) in-vivo dissolution tests according to some embodiments of the present disclosure.

### DETAILED DESCRIPTION OF SPECIFIC EMBODIMENTS

Examples are described herein in the context of drug delivery vehicles as pills or capsules. Those of ordinary skill in the art will realize that the following description is illustrative only and is not intended to be in any way limiting. Reference will now be made in detail to implementations of examples as illustrated in the accompanying drawings. The same reference indicators will be used throughout the drawings and the following description to refer to the same or like items.

In the interest of clarity, not all of the routine features of the examples described herein are shown and described. It will, of course, be appreciated that in the development of any such actual implementation, numerous implementation-specific decisions must be made in order to achieve the developer's specific goals, such as compliance with application- and business-related constraints, and that these specific goals will vary from one implementation to another and from one developer to another.

### I. Introduction

The present disclosure describes a number of embodiments related to capsules, coatings for capsules, methods of producing capsules, and methods of coating capsules. In some embodiments, the present disclosure relates to ingestible capsules to deliver a payload to some portion of a lining of a gastrointestinal tract of the subject. The capsules described herein prevent moisture and vapor ingress into the capsule during delivery of the payload. For example, the capsule may include a moisture-barrier layer (e.g., a parylene layer) that prevents moisture and vapor from entering interior volume of the capsule until delivery of the payload in the gastrointestinal tract. The capsules described herein may also include an enteric coating that leaves little or no residue when dissolved. The friability of the enteric coating of the capsule advantageously allows for mechanical actuators to effectively deploy from the interior volume of the capsule.

In some embodiments, the capsules described herein encapsulate a needle delivery system comprising microneedles. Microneedles are tiny projections of micrometer dimensions and have the capability of delivering drugs, vaccines, and other biomolecules to skin. For example, the microneedles may be dissolvable microneedles that include active pharmaceutical ingredients (APIs). This transdermal delivery platform has many advantages over conventional subcutaneous and intramuscular injection by needle and syringe. First, there is no or minimal pain, cross-infection, and needle stick injuries. Second, microneedles can be designed to target a specific layer of skin. Third, there is potential for self-administration. Last but not least, it can be used when there is a significant first-pass effect of the liver that can prematurely metabolize drugs. Microneedle arrays are usually made of silicon, metals and polymers. Among them, polymer microneedle arrays are increasingly attractive because they are expected to be less expensive to mass produce than silicon or metal arrays and safer during application. Drugs and biomolecules can be incorporated into the interior of microneedles themselves when using dissolving polymers. During application, the polymer structure rapidly dissolves in skin, thereby releasing the drug and biomolecules, so there is no sharp waste.

Capsules are designed to deliver a payload to a region of the subject. For example, during delivery to the gastrointestinal tract, the payload needs to remain stable to ensure efficacy and patient safety. The payload may be affected by moisture which can negatively affect the physical and chemical properties of the payload. For example, dissolvable microneedles that include APIs must be protected from moisture during gastric transit for deployment in the gastrointestinal tract.

Commercial capsules made of materials such as gelatin, hydroxypropyl methylcellulose (HPMC), and pullulan, do not provide adequate moisture protection. Although these commercial capsules may provide some degree of moisture protection, these capsules are not effective for preventing vapor ingress. Additionally, commercial capsules may swell and disintegrate slowly, thereby interfering with deployment of a payload from the capsule (e.g., mechanical actuators). Therefore, improving the mechanical properties of the delivery vehicle (e.g., capsule) could be beneficial in terms of drug efficacy and design flexibility. Additionally, commercial enteric coatings provide some moisture protection, but are insufficient for ultra-moisture sensitive payloads. Commercial enteric coating also swell and disintegrate slowly, interfering with the deployment of a payload within a capsule. In fact, these coatings leave behind residue which can affect delivery of a payload. For example, a capsule including a needle delivery system may need to rupture and pierce the capsule to deploy the needle delivery system, which can be affected by the residue from the enteric coating.

Various examples of the present disclosure are directed to capsules that prevent moisture ingress and rapidly dissolve leaving little or no residue. The capsules described herein prevent moisture and vapor ingress into the capsule during delivery of the payload. For example, the capsule may include a parylene layer coated with a polymer (e.g., polyvinyl alcohol) that prevents moisture from entering the interior volume of the capsule until delivery in the gastrointestinal tract. The capsules described herein may also include an enteric coating that leaves little or no residue when dissolved. The friability of the enteric coating of the capsule advantageously allows for a payload (e.g., mechanical actuators) to effectively deploy from the interior volume of the capsule. In some embodiments, the capsules includes a compact needle delivery systems that utilizes outwardly expanding mechanical actuation to drive needles into engagement with a lining of a gastrointestinal tract or other body lumen, e.g., to facilitate delivery of therapeutic agents or other payloads through such engagement inside the body of a subject.

### II. Methods of Producing a Capsule

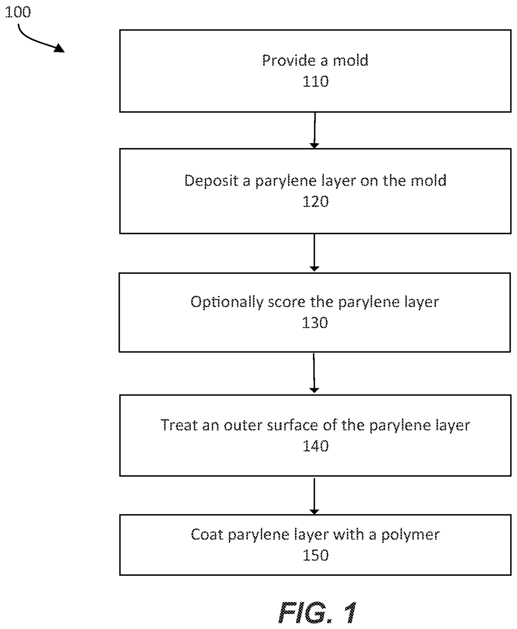

FIG. 1 provides a flow diagram of a method of producing a capsule according to some embodiments. The method 100 may include providing a mold 110. The shape of the mold determines the size and shape of the capsule. The size and shape of mold can be selected to provide a desired shape and volume for the capsule. For example, the mold may comprise a cylinder with a hemispherical end. In this embodiment, two cylinders with hemispherical ends are produced and bonded to form the capsule. In some embodiments, the mold is a finger-like mold that defines the dimensions of the capsule. In some embodiments, the mold comprises a metal mold (e.g., stainless steel mold) or a 3D printed mold. The mold can be coated with a release agent. The release agent prevents adhesion of the capsule on the mold.

In some embodiments, the mold can be a 3D printed mold. The 3D printed mold has a higher surface roughness than a metal mold (e.g., stainless steel), which can lead to problems with release of the capsule from the mold. Due to the surface characteristics of the 3D printed mold, the mold surface may need to be polished for a uniform coating. In some embodiments, the method may include smoothing out the 3D printed mold to reduce the roughness. For example, the mold surface can be polished and cleaned to improve release of the capsule from the mold. In some embodiments, the mold can be polished and coated with a release agent.

The method 100 includes depositing a parylene layer on the mold 120. The parylene layer can be coated or deposited on the mold. For example, the parylene layer can be deposited on the mold using chemical vapor deposition. The parylene layer may form the inner layer of the capsule that prevents moisture ingress into the capsule. In some embodiments, the mold is treated with a release agent prior to depositing the parylene layer. The release agent promotes demolding after the capsule is produced on the mold. In some embodiments, the release agent can be Micro 90. The parylene layer can be deposited on the mold one or more times to tune the thickness of the parylene layer.

In some embodiments, a plurality of parylene layers may be deposited on the mold. For example, the deposition step may comprise a first step comprising depositing a first parylene layer on the mold and a second step comprising depositing a second parylene layer on the first parylene layer. The outer surface of the first parylene layer (e.g., primer layer) can be coated with a release agent prior to the depositing the second parylene layer. In this embodiment, the first parylene layer serves as a release layer from the mold. The two-step deposition of parylene is particularly beneficial for 3D printed molds that may have rough surfaces. The rough surface of the 3D printed mold promotes adhesion of the first parylene layer. In some embodiments, the first parylene serves as a release layer and the second parylene layer serves as a moisture barrier layer. In some embodiments, the metal mold may not utilize a two-stage deposition step.

The method 100 includes optionally scoring the parylene layer 130. After deposition of parylene layer on the mold, the parylene layer can be pre-scored in a desired pattern. The scored parylene layer allows more efficient rupture of the parylene layer for release of a payload (e.g., a needle delivery system). In some embodiments, the parylene layer can be scored with a razor or the parylene layer can be laser-cut into a scoring pattern. For example, the parylene layer can be scored on opposing sides of the capsule. The scoring lines on the parylene layer are beneficial for rupturing the parylene layer when mechanical actuators are delivered to the gastrointestinal tract. The parylene layer is mechanically strong, so the actuation force may not rupture the parylene layer without the scoring lines.

The method 100 includes treating an outer surface of the parylene layer 140. For example, the parylene layer can be treated with an etching solution. In some embodiments, the surface of the parylene layer is treated with oxygen for plasma activation. Treating the surface of the parylene layer with oxygen plasma reverses the polarity of plastic materials (e.g., parylene) and allow the polymers to receive new surface functionalities by increasing the surface tension and creating very small contact angles on the treated materials' surface. This promotes adhesion with any coatings on the parylene layer for good moisture protection properties. Oxygen plasma activation can be performed on the surface of the parylene layer to promote good adhesion with a polymer coating (e.g., polyvinyl alcohol). The adhesion between the parylene layer and the polymer layer has an impact on the moisture barrier properties of the capsule.

The method 100 includes coating the parylene layer with a polymer 150. For example, the parylene surface can be etched with plasma and a polymer can be coated on the etched surface of the parylene layer. In some embodiments, the parylene layer is dip coated in a polymer solution. For example, the parylene layer can be dip coated in a solution comprising polyvinyl alcohol. In some embodiments, the mold is baked to dry the polymer coating on the parylene layer. The parylene layer can be coated with a polymer solution a plurality of times to adjust the coating thickness of the polymer layer. In some embodiments, the mold is dip coated at an elevated temperature to facilitate drying. The speed, viscosity, and number of dips into polymer solution can tune the thickness of the polymer layer.

In some embodiments, the method may include coating the parylene layer in a first polymer solution (e.g., polyvinyl alcohol solution) one or more times for a desired thickness of a first polymer layer. The mold can be baked after each coating of the first polymer solution to form the first polymer layer on the parylene layer. The method may include coating the first polymer layer in a second polymer solution (e.g., polyvinylpyrrolidone solution) one or more times for a desired thickness of a second polymer layer. The mold can be baked after each coating of the second polymer solution to form the second polymer layer on the first polymer layer. In some embodiments, the capsule may comprise a parylene layer and a polyvinyl alcohol layer disposed on the parylene layer. In some embodiments, the capsule may comprise a parylene layer, a polyvinyl alcohol layer disposed on the parylene layer, and a polyvinylpyrrolidone layer disposed on the polyvinyl alcohol layer.

In some embodiments, the polymer solution may comprise biodegradable and bioabsorbable polymers. In some embodiments, the polymer solution comprises natural polymers such as arginine, chitosan, dextrin, polysaccharides, poly (glycolic acid), poly (lactic acid), and hyaluronic acid. In some embodiments, the polymer solution comprises synthetic polymers such as poly (2-hydroxyethyl methacrylate), poly(N-isopropyl acrylamide)s, poly(ethylenimine)s, dendritic polymers, polyvinyl alcohol, or polyvinylpyrrolidone. In some embodiments, the polymer solution comprises a polyvinyl alcohol and polyethylene glycol co-polymer such as Kollicoat IR^{®}.

To prevent the capsule from over-drying and becoming brittle from the plurality of coating and baking steps, the method may include a rehydration step. In some embodiments, the mold can be rehydrated after a plurality of coating and baking steps for forming the polymer layer. The rehydration step prevents the capsule from drying and becoming brittle. The rehydration step may comprise hydrating the capsule in a high humidity environment. In some embodiments, the capsule is rehydrated at a temperature from 10° C to 40° C at a relative humidity ranging from 50% to 95%. In some embodiments, the capsule is rehydrated at a temperature of about 25° C at a relative humidity of about 80%. Rehydrating the capsule at high humidity also softens the capsule for improved demolding. If the capsule gets too moist from the rehydration step, the capsule loses its rigidity and becomes floppy. Therefore, the method may include baking the capsule to regain its structural integrity. For example, the baking step allows the capsule to regain its mechanical structure. The capsule can be removed from the mold after the final baking step. For example, after the polymer layer is coated on the parylene layer, the formed portion of the capsule is removed from the mold. In some embodiments, the capsule is coated with additional polymers before demolding. For example, the capsule can be coated with one or more enteric coatings.

In some embodiments, a method is provided for producing a capsule. The method includes providing a mold. The method includes depositing parylene on the mold to produce a parylene layer. The method includes scoring the parylene layer. The method includes activating the surface of the parylene layer with oxygen plasma. The methods includes dip coating the mold in a polymer solution and baking the dip-coated capsule to produce a polymer layer. In some embodiments, the polymer solution comprises polyvinyl alcohol. In some embodiments, the method includes coating the parylene layer with a plurality of polymer coatings. The method may include coating the capsule with one or more enteric coatings.

It should be appreciated that the specific steps illustrated in FIG. 1 provide a particular method of producing a capsule according to some embodiments. Other sequences of steps may also be performed according to alternative embodiments. For example, alternative embodiments of the present invention may perform the steps outlined above in a different order. Moreover, the individual steps illustrated in FIG. 1 may include multiple sub-steps that may be performed in various sequences as appropriate to the individual step. Furthermore, additional steps may be added or removed depending on the particular applications. One of ordinary skill in the art would recognize many variations, modifications, and alternatives.

In some embodiments, the method for producing a capsule may include melting and reflowing polymers to produce one or more layers of the capsule. The melt and reflow process can be used in combination with dip coating or can be used in lieu of dip coating. For example, a first layer of the capsule can be produced by dip coating into a polymer solution and a second layer of the capsule can be produced by melting and reflowing a polymer (e.g, onto the dip coated layer). The method may include melting and reflowing one or more polymers to produce one or more layers of the capsule. In some embodiments, melting and reflowing polymers to produce a layer of the capsule is particularly suitable for producing thermoplastic capsule materials which are difficult to dissolve in water or solvent for dip coat applications. Additionally, the method including melting and reflowing is a faster and more scalable process for high throughput production of capsules compared to dip coating capsules since it can avoid the time-consuming drying process. In some embodiments, the materials for producing the capsule may include any thermoplastic polymer, a blend of thermoplastic polymers, or a blend of thermoplastic polymers with thermosetting materials suitable for capsules.

In some embodiments, a method for producing a capsules includes wrapping a polymer around a mandrel or mold to produce an assembly, inserting a portion of the assembly into a heat-shrink tube, plugging an end of the assembly with a fixture to define a portion of the exterior of the polymer layer, applying heat to the assembly such that the polymer melts and reflows to conform to the shape of the capsule and the fixture, withdrawing the mandrel and the fixture from each end of the formed capsule, removing the heat shrink wrap, and optionally trimming the length of the capsule. In some embodiments, the mandrel or mold is coated with a parylene layer prior to wrapping the polymer around the mandrel or mold.

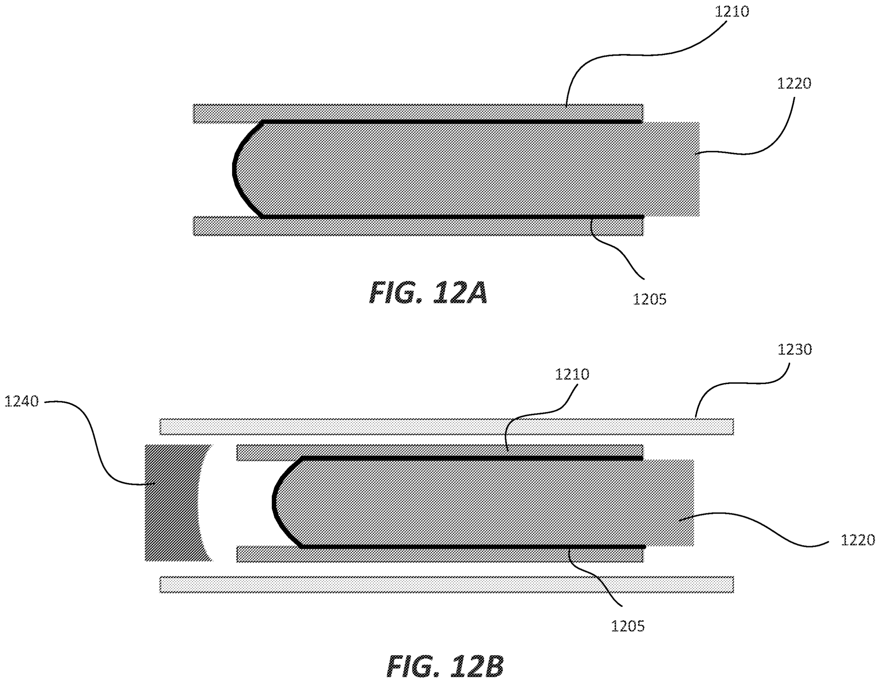

FIGS. 12A-12F show an exemplary process of producing capsules including melting and reflowing polymers to produce one or more layers according to some embodiments of the present disclosure. FIG. 12A shows a polymer film 1210 disposed on a mandrel 1220. The mandrel 1220 may include a parylene layer 1205. In some embodiments, the mandrel 1220 is coated with parylene to form the parylene layer 1205, for example, by chemical vapor deposition. The polymer film 1210 can be wrapped around the parylene layer 1205 and the mandrel 1220 to produce an assembly. The dimensions of the mandrel 1220 correspond to the interior volume of the resulting capsule. The shape of the mandrel 1220 determines the size and shape of the capsule. The size and shape of mandrel 1220 can be selected to provide a desired shape and volume for the capsule. For example, the mandrel 12220 may comprise a cylinder with a hemispherical end. In this embodiment, two cylinders with hemispherical ends are produced and bonded to form the capsule.

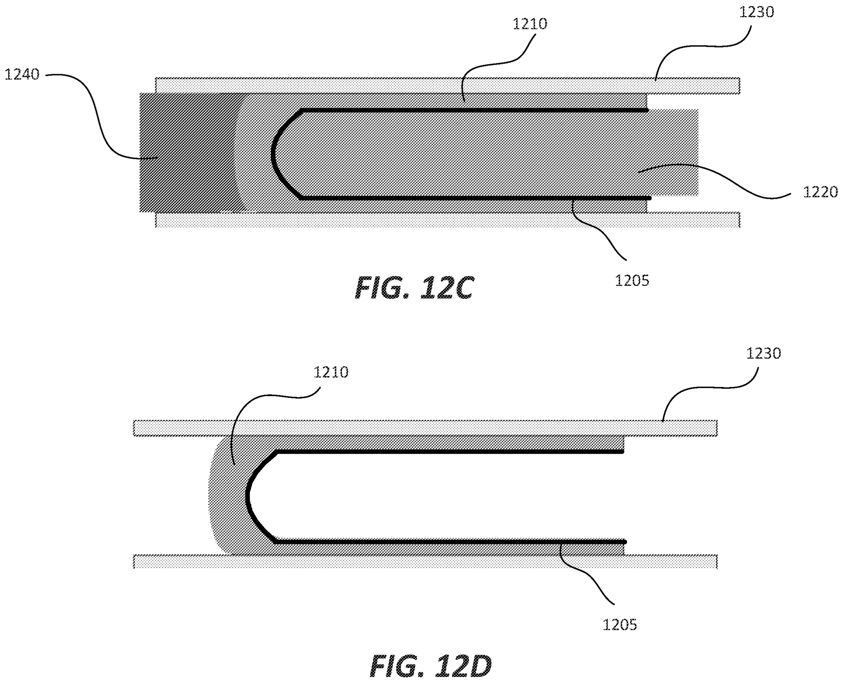

FIG. 12B shows a heat-shrink tube 1230 disposed around the polymer film 1210. The heat shrink tube 1230 can be disposed around the exterior surface of the polymer film 1210. A fixture 1240 can be inserted into one of end of the assembly. The fixture 1240 can define the exterior dimensions of the capsule (e.g., the hemispherical head of a capsule). The fixture 1240 plugs an end of the assembly to define a portion of the exterior of the capsule. For example, FIG. 12B shows a fixture 1240 having a concave end. The concave end may correspond to the dome-shaped end of a capsule. Subsequently, heat can be applied to the assembly to melt and reflow the polymer film 1210. The polymer film 1210 melts and fills the space between the mandrel 1220 and the fixture 1240. As shown in FIG. 12C, the polymer film 1210 conforms with the dimensions of the mandrel 1220 and the fixture 1240 to produce the shape of the capsule. For example, heat can be applied to the polymer film 1210 to melt the polymer which flows in the space between the mandrel 1220 and the fixture 1240. In this way, the polymer conforms to the size and shape of the mandrel 1220 and fixture 1240.

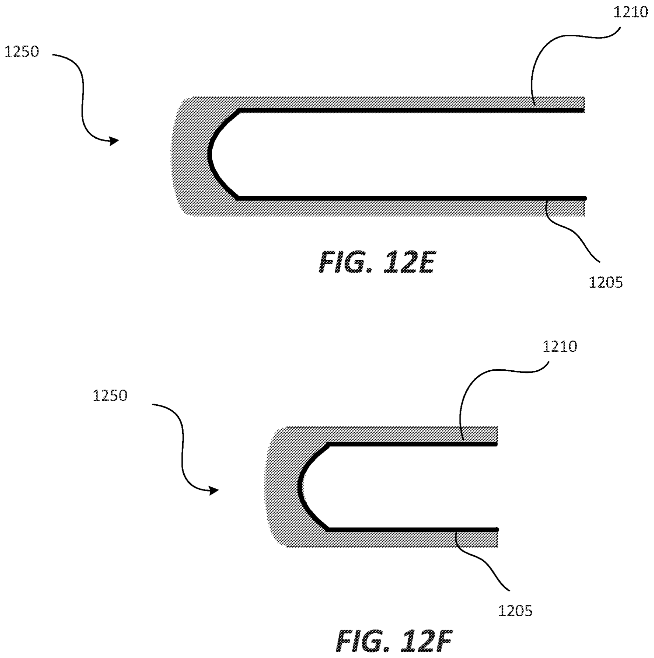

As shown in FIG. 12D, the mandrel 1220 and fixture 1240 can be removed from each end of the assembly after the polymer film 1210 dries to produce the formed capsule 1250 with the heat shrink tube 1230. FIG. 12E shows the heat-shrink tube 1230 removed from the polymer layer to produce the final capsule 1250 including a polymer layer 1210 and a parylene layer 1205. In some embodiments, the length of the capsule 1250 can be trimmed to provide a final length as shown in FIG. 12F.

### III. Capsule Design

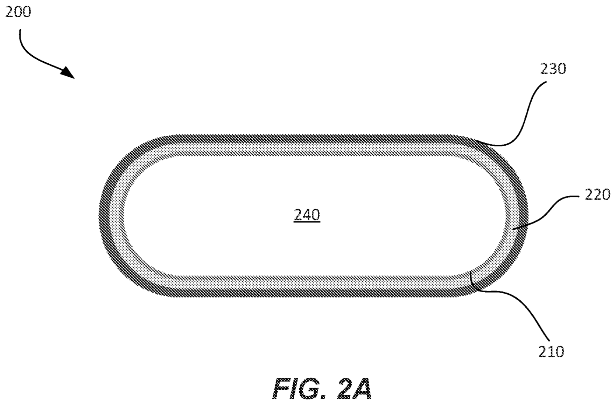

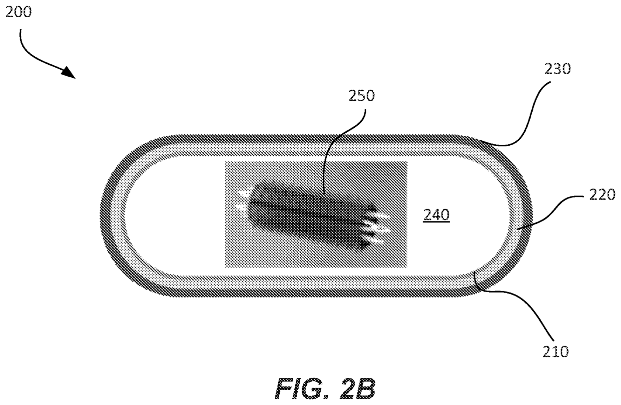

FIGS. 2A and 2B illustrate a capsule according to some embodiments of the present disclosure. In some embodiments, the capsule 200 can be produced according to the methods described herein. The capsule 200 may include a first layer 210, a second layer 220, and a third layer 230. The capsule 200 may be shaped to accommodate a payload in the interior volume 240. For example, the capsule 200 may be cylindrical with opposing hemispherical ends as shown in FIG. 2A. The capsule 200 may be spherical, rectangular, cylindrical, sphero-cylindrical, oval, or any combination thereof.

The first layer 210 may be the inner-most layer of the capsule 200. The first layer 210 may serve as a moisture-barrier layer that protects the contents in the interior volume 240 from moisture and vapor ingress. In some embodiments, the moisture-barrier layer may comprise a polymer. For example, the first layer 210 may comprise a parylene layer. The first layer 210 may have a thickness that provides adequate moisture protection while being sufficiently thin to rupture during delivery of a payload. In some embodiments, the thickness of the first layer 210 may range from 0.5 microns to 30 microns, e.g., from 1 micron to 25 microns, from 2 microns to 20 microns, from 3 microns to 15 microns, from 4 microns to 12 microns, from 5 microns to 10 microns, or from 6 microns to 8 microns. In some embodiments, the thickness of the first layer 210 may range from 5 microns to 10 microns. It was found that a parylene layer having a thickness greater than 30 microns was too thick to rupture and a parylene layer having a thickness less than 0.5 microns did not provide adequate moisture protection.

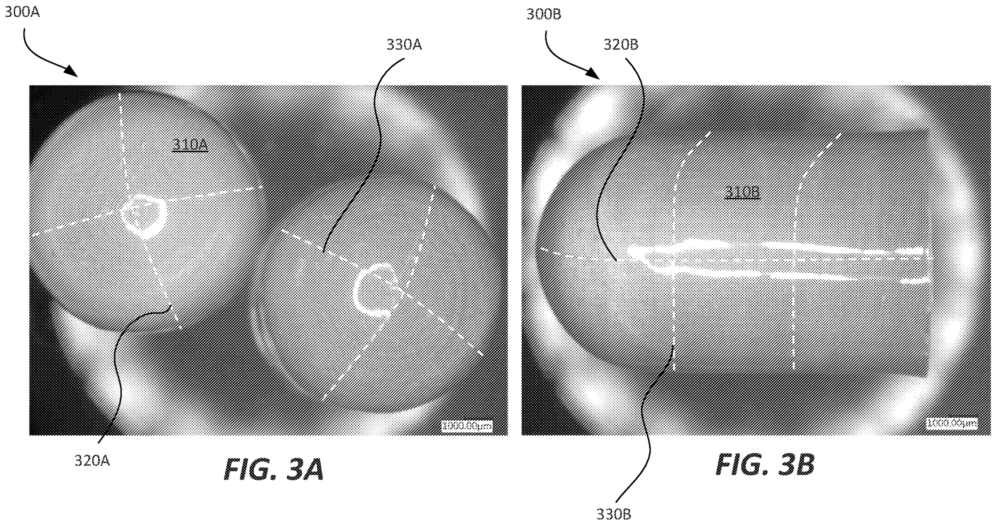

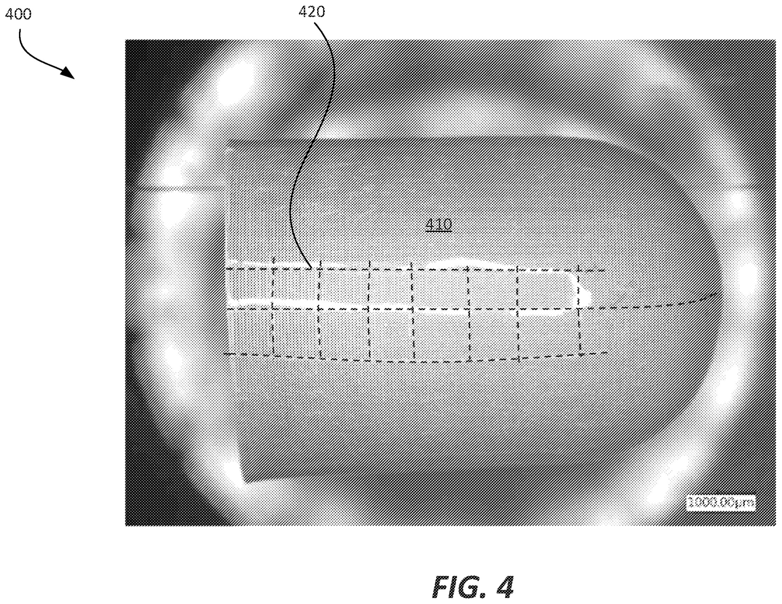

In some embodiments, the first layer 210 is scored or pre-cut to form rupture lines. For example, the first layer 210 may be cut with razor blade or laser cut. The first layer 210 may be pre-cut into a pattern that promotes rupture by the payload in the capsule 200. As shown in FIGS. 3A and 3B, the parylene layer of the capsule is scored to form rupture lines. FIG. 3A shows a scoring pattern including a first score line 320A and a second score line 330A on the parylene layer 310A of the capsule 300A. In this embodiment, the first score line 320A and the second score line 330A intersect at distal ends of the parylene layer 310A of the capsule 300A (e.g., the hemispherical ends of the capsule). FIG. 3B shows an alternative scoring pattern for the parylene layer 310B of the capsule 300B. The parylene layer 310B may include a first score line 320B along the longitudinal axis of the capsule 300B and a plurality of second score lines 330B that intersect the first score line 320B. FIG. 4 shows the parylene layer 410 of the capsule 400 scored into a grid pattern 420. The parylene layer 410 may include the scored grid pattern on a plurality of portions of the parylene layer 410. For example, the parylene layer 410 may include the grid pattern 420 on opposing sides of the capsule 400. The score lines on the parylene layer can be selected to promote rupture based on the size, function, and properties of the payload.

In some embodiments, a second layer 220 is disposed over the first layer 210. For example, the second layer 220 can be deposited or coated onto the first layer 210. In some embodiments, the capsule 200 comprising the first layer 210 may be dip-coated in a polymer solution to form the second layer 220. In some embodiments, the second layer 220 may comprise a polymer, collagen, fatty acids, starch, gelatin, pullulan, or hydroxypropyl methylcellulose. In some embodiments, the polymer may be biodegradable and bioabsorbable polymers. In some embodiments, the polymer comprises natural polymers such as arginine, chitosan, dextrin, polysaccharides, poly (glycolic acid), poly (lactic acid), and hyaluronic acid. In some embodiments, the polymer comprises synthetic polymers such as poly (2-hydroxyethyl methacrylate), poly(N-isopropyl acrylamide)s, poly(ethylenimine)s, dendritic polymers, polyvinyl alcohol, or polyvinylpyrrolidone.

In some embodiments, the second layer 220 comprises polyvinyl alcohol. The surface of the parylene layer contacting the second layer 220 is treated before coating with polyvinyl alcohol. For example, the surface of the parylene layer can be treated with oxygen plasma to roughen the surface of the parylene layer to promote adhesion with the polyvinyl alcohol.

In embodiments where the first layer 210 comprises parylene, it was found that a second layer 220 comprising polyvinyl alcohol synergistically bonds to the parylene to form a moisture barrier. Additionally, it was found that a capsule including a first layer comprising parylene and a second layer comprising polyvinyl alcohol had improved demolding properties. In particular, the mechanical toughness and ductility of parylene coated with polyvinyl alcohol were excellent for removing the capsule from the mold without any breakage.

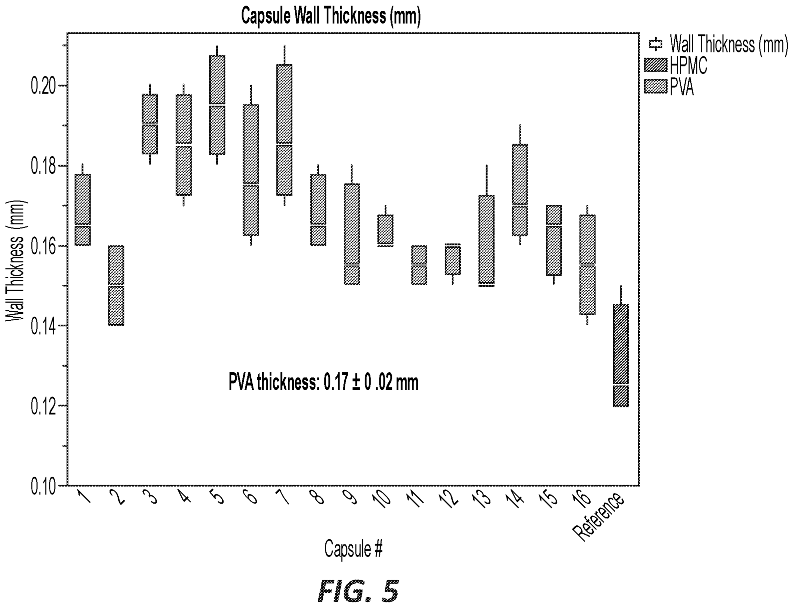

FIG. 5 shows a graph of the thickness of the second layer 220 according to some embodiments. The graph shows the thickness range of a polyvinyl alcohol layer. The thickness of the second layer 220 may range from 100 microns to 250 microns, e.g., from 220 microns to 240 microns, from 240 microns to 230 microns, from 125 microns to 220 microns, from 130 microns to 210 microns, from 140 microns to 200 microns, from 150 microns to 190 microns, or from 160 microns to 180 microns. In some embodiments, the thickness of the second layer 220 may range from 150 microns to 190 microns.

In some embodiments, the capsule 200 may include a third layer 230. The third layer may comprise an enteric coating. The enteric coating may comprise a polymer that prevents dissolution or disintegration in the stomach (e.g., in a gastric environment). The enteric coating may protect the payload in the interior volume 240 of the capsule 200 from the acids in the stomach. In some embodiments, the enteric coating may comprise anionic methacrylate copolymer (e.g., EUDRAGIT^{®} L 100-55) and plasticizer. The plasticizer can be one or more of alkyl citrates, glycerol esters, alkyl phthalates, alkyl sebacates, sucrose esters, sorbitan esters, diethyl sebacate, dibutyl sebacate, propylenglycol and polyethylene glycols, triethyl citrate, acetyl triethyl citrate, diethyl sebacate, or dibutyl sebacate. In some embodiments, the plasticizer may be dibutyl sebacate.

In some embodiments, the third layer 230 may comprise a first sublayer and a second sublayer. For example, the third layer may comprise a first sublayer comprising a polymer, talc, and a plasticizer and a second sublayer comprising a polymer and a plasticizer. In some embodiments, the first sublayer comprises anionic methacrylate copolymer (e.g., EUDRAGIT^{®} L 100-55), talc, and plasticizer (e.g., dibutyl sebacate). In some embodiments, the second sublayer comprises anionic methacrylate copolymer (e.g., EUDRAGIT^{®} L 100-55) and plasticizer (e.g., dibutyl sebacate). The second sublayer may be coated onto the first sublayer.

The capsule 200 may comprise an interior volume 240 to receive a payload. In some embodiments, the payload may comprise an API, a mechanical actuator, a biomedical device (e.g., an implantable pulse generator), or combinations thereof. For example, FIG. 2B shows a capsule 200 including a mechanical actuator 250 as the payload. The mechanical actuator 250 may include a needle delivery system according to some embodiments of the present disclosure. In the illustrative example, a subject may wish to take a dose of an API or other compound without resorting to a syringe injection, intravenous infusion, and potential accompanying discomfort or other concerns. To this end, the person may take a capsule according to this disclosure to provide a dosage of an API or a needle system comprising the API. For example, the drug delivery vehicle can be pill or capsule form that the subject can swallow. In some embodiments, the drug delivery vehicle may include components capable of deploying within the body to effectively provide an internal injection, which may cause much smaller tissue disturbances and fewer systemic effects as compared to an external injection or infusion. As the drug delivery vehicle reaches a target portion of the gastrointestinal tract (such as the duodenum), a specialized coating of the pill or capsule has dissolved or degraded sufficiently to break apart and allow a mechanical actuator within the pill to expand outwardly.

In use, the capsule 200 may travel to a target portion of the gastrointestinal tract (such as the duodenum), and a coating of capsule may dissolve or degrade sufficiently to break apart and allow a mechanical actuator within the capsule to expand outwardly. Various options may be employed for the outwardly expanding mechanical actuators, such as a normally- or radially-expanding stent-like tube, an unwinding coil, an unfurling set of curved arms, a set of double-hinged arms that unfold relative to a central hub and then unfold again relative to parts connected to the hub, or a scissor-lift-like arrangement with centrally-hinged lateral columns that pop upright to an expanded state from a compressed state in which portions of the columns are hinged toward each other.

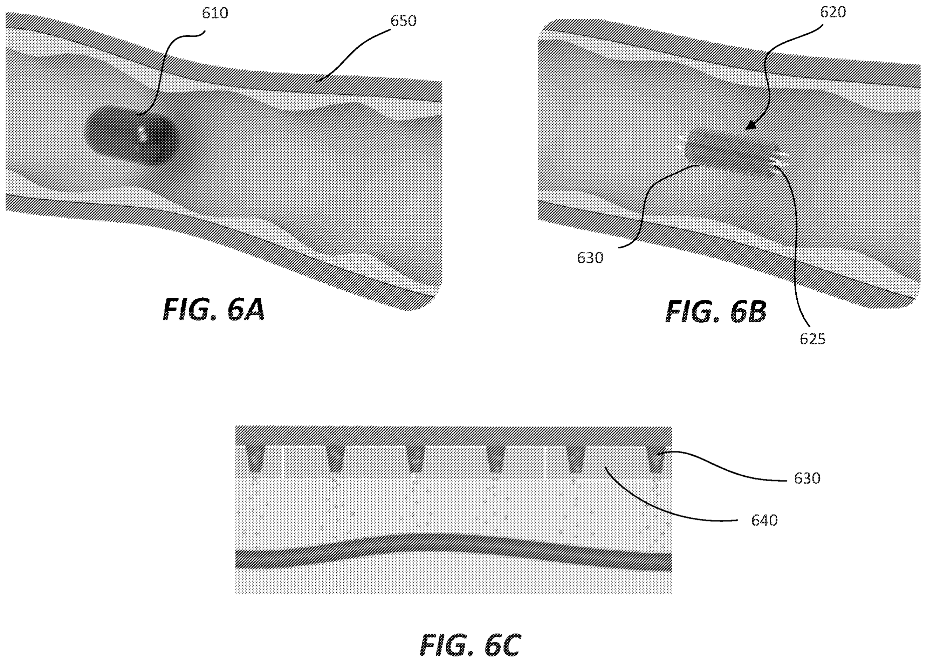

FIG. 2B shows multiple arrays of microneedles arranged about the mechanical actuator. The multiple arrays of microneedles can be driven by outward expansion into engagement with surrounding tissue (e.g., tissue of a mucosal lining of the duodenum). The drug dose can be delivered to the tissue through the engaged microneedles, such as by flowing through the microneedles if hollow or by direct absorption if the drug is embedded in a dissolvable composition of the microneedles (FIG. 6C). After delivery of the dosage, the constituent parts of the device may biodegrade and avoid possible complications from trying to pass the device remains out of the body. Thus, the subject may use the device to administer an internal injection that may ultimately be less invasive, less arduous, and/or less troublesome to the subject than the alternative of using an external syringe or intravenous infusion.

### IV. Method of Delivering Capsule to the Gastrointestinal Tract

FIGS. 6A-6C illustrates a method for delivering a payload to a target region of a subject using a capsule according to some embodiments of the present disclosure. In this embodiment, a mechanical actuator is deployed from a capsule; however, the capsule is effective for delivering any payload described herein. FIG. 6A shows a capsule 610 traveling through a portion of the gastrointestinal tract 650. The capsule 610 may comprise a plurality of layers. In some embodiments, the capsule comprises an innermost layer comprising parylene, a polymer layer disposed on the innermost layer, and an enteric coating disposed on the polymer layer. The polymer layer may comprise polyvinyl alcohol and the enteric coating may comprise anionic methacrylate copolymer (e.g., EUDRAGIT^{®} L 100-55), talc, and a plasticizer. In some embodiments, an overcoat may be applied to the enteric coating layer comprising anionic methacrylate copolymer (e.g., EUDRAGIT^{®} L 100-55) and plasticizer. The polymer layer and the enteric coating can be tuned to a specific thickness for deployment in a specific region of the gastrointestinal tract.

In some embodiments, the capsule 610 may comprise a parylene layer, one or more polymer layers, and, optionally, one or more enteric coatings. For example, the capsule may comprise an inner layer comprising parylene, a second layer comprising polyvinyl alcohol, a third layer comprising polyvinylpyrrolidone, and an enteric coating. Although polyvinyl alcohol is water soluble, polyvinylpyrrolidone dissolves faster than polyvinyl alcohol. However, the parylene layer in combination with a polyvinyl alcohol layer achieves the best release from the mold. This is because polyvinyl alcohol is mechanically tough and flexible so it does not break when lifting from mold. The choice of base capsule material is key for good release from mold as the parylene layer is too brittle with other materials. As described above, the capsule described herein comprises an enclosure including an inner layer comprising parylene and an outer layer comprising a polymer. The parylene inner layer provides a moisture barrier, thereby protecting the contents of the drug delivery vehicle.

FIG. 6B shows the payload 620 deployed from the capsule 610. The capsule 610 releases a payload 620 after being transferred to the intestine without releasing the payload in the stomach under a strongly acidic environment. The capsule 610 protects the payload from gastric acid or gastric enzymes or to cause the payload 620 to be released through use of a period of time during which the capsule is transferred from the stomach to the small intestine. In some embodiments, the payload 620 may include a mechanical actuator 625 within the interior volume of the capsule 610. The mechanical actuator 625 may comprise a needle delivery system comprising a plurality of microneedles 630. The microneedles 630 may include an active pharmaceutical ingredient.

As shown in FIG. 6C, the mechanical actuator 625 can deploy the microneedles 630 into the gastrointestinal lining. The microneedles 630 pierce the gastrointestinal lining 640 to deliver the active pharmaceutical ingredient in the gastrointestinal tract. In some embodiments, the microneedles 630 are dissolvable microneedles that include biotherapeutics. The microneedles 630 may dissolve after piercing the gastrointestinal tract to release the biotherapeutic as shown in FIG. 6C.

### V. Enteric Coating

The present disclosure provides an enteric coating composition comprising a polymer, talc, and a plasticizer. For example, the enteric coating may include EUDRAGIT^{®} L 100-55 (polymer), talc, and dibutyl sebacate (plasticizer). The enteric coating described herein is designed to remain intact in the stomach and dissolve in the gastrointestinal tract (e.g., the intestine). The enteric coating can be applied to solid dosage forms, such as granules, pellets, capsules, or tablets. The enteric coating protects payload within a capsule from gastric acids in the stomach, and improves drug bioavailability by preventing degradation of acid or gastric enzyme labile drugs. The enteric coating described herein beneficially resists disintegration or dissolution in the stomach, dissolves or disintegrates rapidly in the small intestine, remains physically and chemically stable during storage, and is easily applied as a coating.

Additionally, the enteric coating described herein beneficially prevents moisture and vapor ingress into a drug delivery vehicle (e.g., a capsule). The talc in the enteric coating produces an enteric coating layer that is friable. The friability of the enteric coating results in an enteric coating that rapidly dissolves and surprisingly does not leave any residue after dissolution. This is particularly advantageous for payloads including mechanical actuators as the enteric coating does not interfere with deployment of mechanical actuators.

In some embodiments, the enteric coating comprises a copolymer. The copolymer can be an anionic methacrylate copolymer polymerized from of 40 to 60 wt. % by weight methacrylic acid and 60 to 40 wt. % methyl methacrylate or 60 to 40 wt. % ethyl acrylate. For example, the copolymer may be polymerized from 50 wt. % ethyl acrylate and 50 wt. % methacrylic acid. Commercially available anionic methacrylate copolymers that may be used in the enteric coating composition described herein may include EUDRAGIT^{®} L 100-55 or EUDRAGIT^{®} L (available from Evonik Industries, Essen, Germany).

In some embodiments, the anionic methacrylate copolymer may be present in the enteric coating in an amount ranging from 20 wt. % to 60 wt. %, based on the total weight of the enteric coating. For example, the anionic methacrylate copolymer may be present in the enteric coating in an amount ranging from 25 wt. % to 55 wt. %, from 30 wt. % to 55 wt. %, from 30 wt. % to 40 wt. %, from 35 wt. % to 55 wt. %, from 35 wt. % to 50 wt. %, from 35 wt. % to 45 wt. %, or from 40 wt. % to 50 wt. %, based on the total weight of the enteric coating.

In some embodiments, talc may be present in the enteric coating in an amount ranging from 40 wt. % to 80 wt. %, based on the total weight of the enteric coating. For example, talc may be present in the enteric coating in an amount ranging from 45 wt. % to 80 wt. %, from 45 wt. % to 75 wt. %, from 50 wt. % to 75 wt. %, from 50 wt. % to 70 wt. %, from 55 wt. % to 65 wt. %, or from 60 wt. % to 70 wt. %, based on the total weight of the enteric coating. It was found that adding talc improves friability of the enteric coating. However, talc is water-insoluble making the enteric coating more brittle. If talc is included in the enteric composition in amounts greater than 80 wt. %, the enteric coating becomes too brittle. If talc is included in the enteric coating in amounts less than 40 wt. %, the enteric coating does not achieve sufficient friability. An enteric coating including talc in an amount ranging from 40 wt. % to 80 wt. %, based on the total weight of the enteric coating, provides a balance of friability and ductility for the enteric coating.

The ratio of talc to anionic methacrylate copolymer in the enteric coating may range from 0.5:1 to 10:1, e.g., from 1:1 to 8:1, from 1.25:1 to 6:1, from 1.5:1 to 4:1, from 1:1 to 3:1, from 1:1 to 2:1, or from 1.25:1 to 1.75:1. In some embodiments, the ratio of talc to anionic methacrylate copolymer is 1.5:1. The talc in combination with anionic methacrylate copolymer produces a friable layer. Advantageously, an enteric coating comprising anionic methacrylate copolymer and talc dissolves rapidly and leaves little or no residue. The dissolution properties and minimal amount of residue beneficially does not interfere with deployment of a payload within a capsule.

In some embodiments, a plasticizer may be present in the enteric coating in an amount ranging from 5 wt. % to 25 wt. %, based on the total weight of the polymer (e.g., the anionic methacrylate copolymer). For example, the plasticizer may be present in the enteric coating in an amount ranging from 5 wt. % to 20 wt. %, from 5 wt. % to 15 wt. %, from 5 wt. % to 10 wt. %, from 10 wt. % to 20 wt. %, or from 10 wt. % to 15 wt. %, based on the total weight of the polymer. The plasticizer may be hydrophobic. In some embodiments, the plasticizer comprises dibutyl sebacate.

The enteric coating composition (e.g., polymer, talc, and plasticizer) may be provided in a solvent mixture to produce an enteric coating solution. A drug delivery vehicle can be coated in the enteric coating solution and dried to produce an enteric coating layer. The solvent may comprise one or more of acetone, isopropyl alcohol, and water. The solvent mixture may comprise a solids (e.g., polymer) content from 20 wt. % to 50 wt. %, e.g., from 20 wt. % to 45 wt. %, from 20 wt. % to 40 wt. %, from 25 wt. % to 40 wt. %, from 30 wt. % to 40 wt. %, or from 30 wt. % to 35 wt. %. For example, the solids in the solvent mixture may comprise polymer, talc, and plasticizer.

In some embodiments, the present disclosure provides a method of producing an enteric coating composition. The method of producing a coating composition may include providing a solvent. The solvent may comprise water, acetone, and isopropyl alcohol. In some embodiments, the solvent may include a plasticizer. The plasticizer can be added to the solvent. For example, the plasticizer can be mixed in the solvent mixture. The method includes adding anionic methacrylate copolymer and talc to the solvent to produce the coating composition. In some examples, the anionic methacrylate copolymer and talc can be mixed together prior to adding the components to the solvent. For example, the anionic methacrylate copolymer and talc can be mixed in a solvent (e.g., water, acetone, and/or isopropyl alcohol) and then added to the solvent comprising the plasticizer. The enteric coating composition described herein can be coated on a drug delivery vehicle (e.g., a capsule).

### VI. Methods of Coating Capsule with an Enteric Coating

According to some embodiments, the present disclosure provides a method for coating a capsule with an enteric coating. The method includes coating a capsule with an enteric solution to produce a first enteric coating layer. The first enteric coating layer may comprise an anionic methacrylate copolymer (e.g., EUDRAGIT^{®} L 100-55), talc, and plasticizer. In some embodiments, the method may also include coating the first enteric coating layer with a second enteric coating layer. For example, the second enteric coating layer may comprise an anionic methacrylate copolymer (e.g., EUDRAGIT^{®} L 100-55) and plasticizer. The second enteric coating layer may be disposed over the first enteric coating layer to hold the first enteric coating intact. In particular, the second enteric coating layer can provide rigidity to the first enteric coating layer since the first enteric coating layer is friable due to the presence of talc. In some embodiments, the enteric coated capsule may include a payload (e.g., a needle delivery system including a therapeutic).

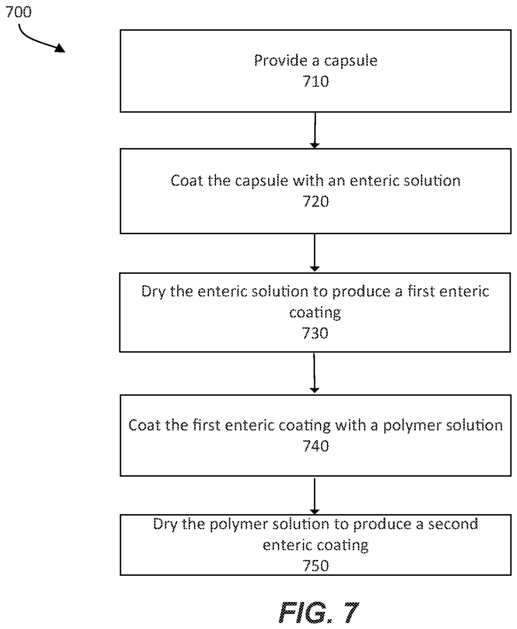

FIG. 7 provides a flow diagram of a method for coating a capsule with an enteric coating according to some embodiments of the present disclosure. The method 700 includes providing a capsule 710. The capsule can be any capsule for delivering a payload. For example, the capsule may comprise a polymer, collagen, fatty acids, starch, gelatin, pullulan, or hydroxypropyl methylcellulose. In some embodiments, the capsule may include first layer comprising parylene and a second layer comprising a polymer as described herein. For example, the capsule may include a polyvinyl alcohol layer coated onto a parylene layer. The parylene layer may include a scored pattern that provides rupture lines for a payload. For example, the payload may include a mechanical actuator. The mechanical actuator can be deployed from the capsule by rupturing the score lines of the parylene layer.

The method 700 may include coating the capsule with an enteric solution 720. In some embodiments, coating the capsule with an enteric solution may include dip coating the capsule in the enteric solution. The enteric solution comprises a solvent, anionic methacrylate copolymer, talc, and plasticizer. In some embodiments, the anionic methacrylate copolymer may be EUDRAGIT^{®} L 100-55. The enteric composition may include talc and anionic methacrylate copolymer in a ratio from 0.5:1 to 10:1. The enteric composition may be provided in a solvent to produce the enteric solution. For example, the enteric composition comprising talc and anionic methacrylate copolymer can be mixed in the ratios described herein and then added to the solvent to produce the enteric solution.

The method 700 includes drying the enteric solution to produce a first enteric coating 730. The drying conditions of the first enteric coating are controlled to provide a uniform enteric coating with no air bubbles. The drying conditions are important to suppress air bubbles in the enteric coating layer to provide a high quality enteric coating. The capsule coated with enteric solution can be partially dried in a rotisserie followed by drying in an oven to form an enteric coating. The rotisserie is configured to rotate the capsule to homogenize the enteric solution and provide a uniform thickness for the enteric coating.

In some embodiments, drying the enteric solution to produce a first enteric coating comprises a plurality of steps. For example, the drying step may include a first drying step comprising drying the enteric coating at ambient conditions in a rotisserie without forced air, a second drying step comprising drying the enteric coating at ambient conditions in the rotisserie with forced air, and a third drying step comprising drying the enteric coating in an oven at an elevated temperature. The first drying step may be ambient drying of the enteric coating without the use of forced air (e.g., no fans). The second drying step may be ambient drying of the enteric coating using forced air (e.g., with fans). The first and second drying step may be partial drying steps. It was found that insufficient partial drying before heat drying (e.g., heated forced air) and/or sudden exposure to high drying temperatures in an oven leads to a lower quality enteric coating with bubbles.

In some embodiments, the drying step may comprise rotating the capsule in a rotisserie at ambient conditions for a predetermined time (e.g., from 30 seconds to 5 minutes) followed by rotating the capsule in a rotisserie at ambient conditions with forced air for a predetermined time (e.g., from 30 seconds to 5 minutes). The capsule can be then heated in an oven to fully dry the enteric coating on the capsule. In some embodiments, the forced air may be heated or ambient air supplied by a fan. The enteric coating process and drying process can be repeated a plurality of times to tune the thickness of the enteric coating. For example, the capsule can be coated with enteric solution and heated in the rotisserie to apply a plurality of coats of enteric coating.

The method 700 includes coating the enteric-coated capsule with a polymer solution 740. The polymer solution may be a second enteric solution. For example, the second enteric solution may comprise anionic methacrylate copolymer (e.g., EUDRAGIT^{®} L 100-55) and plasticizer. In some embodiments, the plasticizer can be one or more of alkyl citrates, glycerol esters, alkyl phthalates, alkyl sebacates, sucrose esters, sorbitan esters, diethyl sebacate, dibutyl sebacate, propylenglycol and polyethylene glycols, triethyl citrate, acetyl triethyl citrate, diethyl sebacate, and dibutyl sebacate. The plasticizer may be present in an amount range from 10 to 60 wt.% (e.g., from 10 to 60 wt.%, from 20 to 60 wt.%, from 25 to 55 wt.%, from 30 to 50 wt.%, from 30 to 60 wt.%, or from 40 to 60 wt.%) , based on the total weight of the polymer. For example, the plasticizer may be present at concentration of about 10 wt. %, 20 wt. %, 30 wt. %, 40 wt. %, 50 wt. %, or 60 wt. %, based on the total weight of the polymer. The method 700 includes drying the polymer solution to produce a second enteric coating 750.

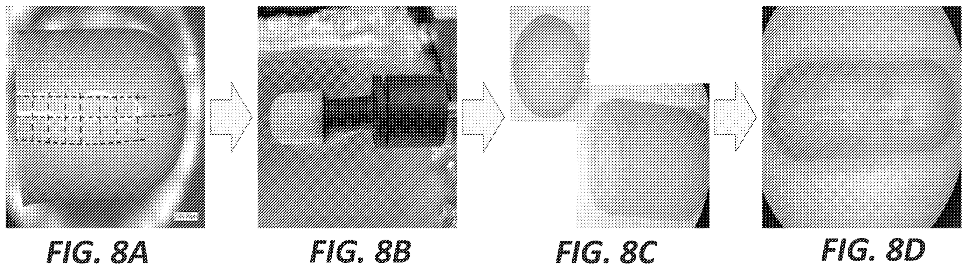

FIGS. 8A-D show a manufacturing process for producing an enteric coated capsule according to some embodiments. FIG. 8A shows a capsule comprising a polyvinyl alcohol layer coated onto a parylene layer. The parylene layer includes a scored pattern that provides rupture lines for the payload. For example, the capsule may include a mechanical actuator as the payload. The mechanical actuator can be deployed from the capsule by rupturing the score lines of the parylene layer.

FIG. 8B shows the process of coating the capsule with an enteric solution. The enteric solution may comprise solvent, a polymer, talc, plasticizer, and water. In some embodiments, the polymer comprises anionic methacrylate copolymer (e.g., EUDRAGIT^{®} L 100-55). The anionic methacrylate copolymer and talc can be mixed in the ratios described herein and then added to the solvent. In some embodiments, coating the capsule with an enteric solution may include dip coating the capsule into the enteric solution.

The capsule coated with enteric solution can be dried in a rotisserie as shown in FIG. 8. The rotisserie is configured to rotate the capsule to homogenize the enteric solution and provide a uniform thickness for the enteric coating. In some embodiments, forming the enteric-coated capsule may include dip coating the capsule in an enteric solution, partial drying of the enteric solution in a rotisserie at ambient conditions for a predetermined time (e.g., from 30 seconds to 5 minutes), partial drying of the enteric solution at ambient conditions in a rotisserie at ambient conditions with forced air for a predetermined time (e.g., from 30 seconds to 5 minutes), and final drying of the enteric solution in an oven at an elevated temperature. In some embodiments, the forced air may be supplied by a fan. The enteric coating process and drying process can be repeated a plurality of times to tune the thickness of the enteric coating.

The drying process advantageously prevents bubble formation in the enteric coating layer. In conventional processes, the evaporation of solvent in the enteric solution is faster than evaporation of water, which leads to trapped air bubbles in the enteric coating layer. Beneficially, the rotisserie homogenizes the enteric coating and provides a uniform thickness of the coating without any air bubbles. In some embodiments, the drying process in the rotisserie comprises partially drying the enteric solution which comprises a first step of rotating the capsule in ambient conditions followed by a second step of rotating the capsule while applying forced air. In some embodiments, the process may include over-coating the enteric coating with another polymer. For example, the enteric coating can be coated with another enteric coating comprising anionic methacrylate copolymer (e.g., EUDRAGIT^{®} L 100-55) and plasticizer.

FIG. 8C shows a process of applying a belt to the capsule. The capsule may comprise two different molded components that form the capsule. To ensure proper jointing of the two molded components to form the capsule, a belt portion may be provided to the open end of the capsule. For example, FIG. 8C shows a polyvinyl alcohol belt attached (e.g., glued) to the open end of the capsule. The belt assists with butt-joint and banding. As shown in FIG. 8D, the butt-joint can be sealed with an enteric coating comprising anionic methacrylate copolymer and talc. In some embodiments, an enteric overcoat can be applied to the butt-joint. The overcoat may comprise anionic methacrylate copolymer and plasticizer. The final capsule may include a scored parylene layer, a polyvinyl alcohol layer, a first enteric coating comprising anionic methacrylate copolymer, talc, and plasticizer, and a second enteric coating comprising anionic methacrylate copolymer and plasticizer. In some embodiments, the anionic methacrylate copolymer is EUDRAGIT^{®} L 100-55. The joint between the two portions of the capsule may be glued using any of combination of the aforementioned enteric coatings.

In some embodiments, the method of coating a capsule includes providing a base capsule and coating the base capsule with an enteric solution. In some embodiments, the base capsule is dip coated in the enteric solution. The method includes drying the enteric solution on the base capsule as described herein. In some embodiments, the capsule is partially dried in a rotisserie followed by drying in an oven. For example, the base capsule coated with the enteric solution is rotated while applying heat or forced air to the capsule. In some embodiments, forced air is supplied to the rotisserie to facilitate drying. In some embodiments, the method includes a plurality of coating and drying steps to build and tune the thickness of the enteric coating. The method includes over-coating the enteric coating of the capsule with a second enteric coating. For example, the base capsule can be coated with a second enteric coating comprising anionic methacrylate copolymer (e.g., EUDRAGIT^{®} L 100-55) and plasticizer.

In some embodiments, the enteric composition comprises talc to anionic methacrylate copolymer (e.g., EUDRAGIT^{®} L 100-55) in a ratio of 60:40. The enteric composition can be a dry powder that is added to a solvent to produce the enteric solution. Adding more talc to the enteric composition improves friability, but talc is insoluble in water making the enteric coating more brittle. The dried enteric coating includes talc embedded in the anionic methacrylate copolymer and plasticizer. In some embodiments, an additional enteric layer (e.g., an overcoat) comprising anionic methacrylate copolymer is coated on the enteric coating.

### VII. Examples

### Dissolution Rates and Gastric Protection of HPMC capsules

Sample capsules were tested to determine moisture ingress and dissolution properties of HPMC capsules. Specifically, soak testing in simulated gastric fluid (SGF) was performed to understand moisture ingress (where enteric layer must protect base capsules from dissolving in the acidic environment as the enteric coating will remain intact), and dissolution testing in simulated intestinal fluid (SIF) or phosphate buffered saline (PBS) was performed to understand enteric-coated capsules dissolution rates and the residue from dissolved capsules. Table 1 provides the sample capsule compositions for Comparative Examples 1-5. Each of the sample capsules included HPMC as the base capsule material.

| TABLE 1 | | | |
|---|---|---|---|
| | Base Capsule Material | Enteric Coating | Inner Layer |
| Comparative Example 1 | HPMC | EUDRAGIT^{®} L 100-55 + DBS | Parylene-C inner coating |
| Comparative Example 2 | HPMC | EUDRAGIT^{®} L 100-55 + DBS | Parylene-C, capsule gap |
| Comparative Example 3 | HPMC - capsule modifications | Thinner EUDRAGIT^{®} L 100-55 + TEC | |
| Comparative Example 4 | HPMC | EUDRAGIT^{®} L100-55 + DBS glue | None |
| Comparative Example 5 | HPMC | Uncoated capsule | none |

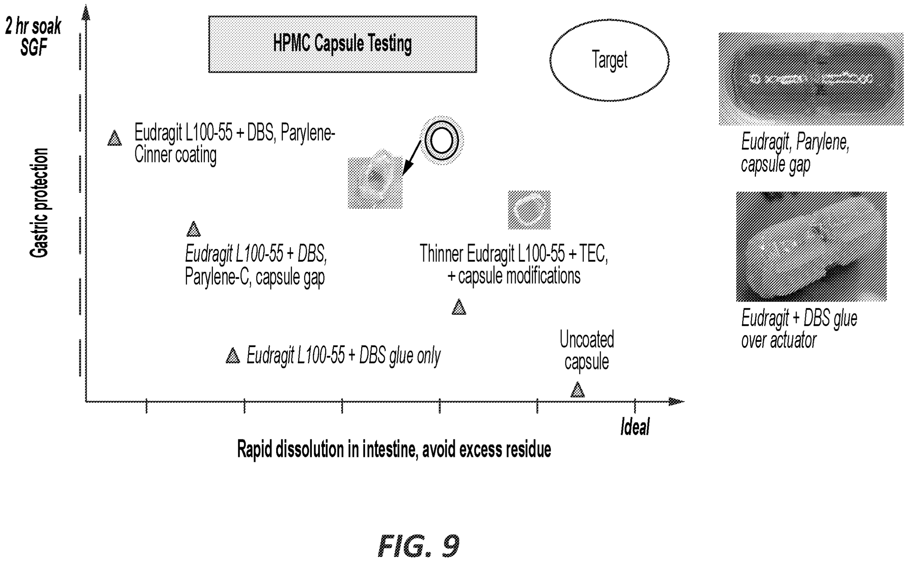

FIG. 9 provides a qualitative analysis of the capsule design on moisture protection and dissolution rates. For example, FIG. 9 shows the effect of the enteric coating layer thickness, plasticizers, and parylene on moisture protection and dissolution rates. As shown in FIG. 9, Comparative Examples 1-5 did not achieve a good combination of rapid dissolution without excess residue and gastric protection. For example, Comparative Examples 1, 2, and 4 did not achieve rapid dissolution and left substantial residue. In fact, Comparative Example 4 left enough residue to prevent the mechanical actuator in the HPMC capsule from deploying. The modified capsule of Comparative Examples 3 and 5 provided good dissolution rates, but did not provide adequate gastric protection (e.g., moisture ingress). Therefore, capsules containing HPMC as the base capsule material did not provide good properties for delivering a payload to the gastrointestinal tract.

### Dissolution Rates and Residue Tests of Capsules

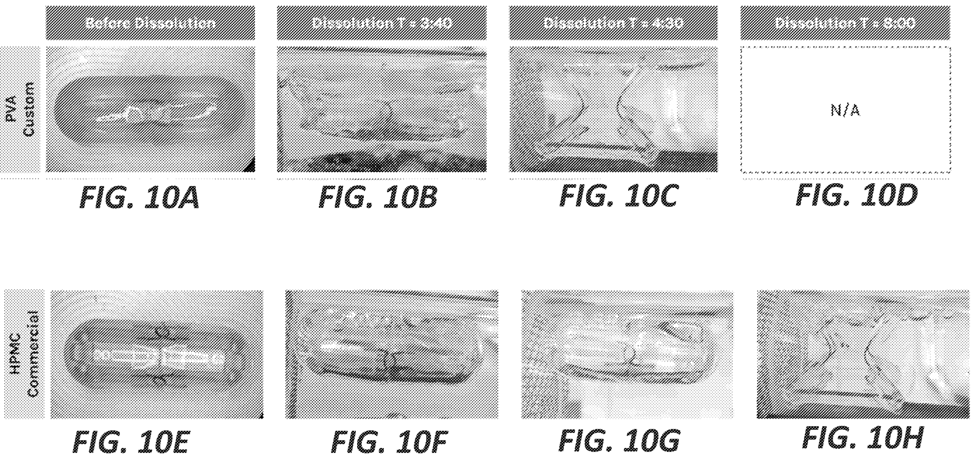

FIGS. 10A-10H provide dissolution tests for different base capsule materials in simulated intestinal fluid at fixed time points. Specifically, FIGS. 10A to 10D show the dissolution rate of polyvinyl alcohol capsules and FIGS. 10E to 10H show the dissolution rate of HPMC capsules. The dissolution rates of the capsules are shown for time stamps before dissolution (FIGS. 10A and 10E), at 3.67 minutes (FIG. 10B and 10F), at 4.5 minutes (FIG. 10C and 10G), and at 8.0 minutes (FIG. 10D and 10H). The tests demonstrate how long it takes for each base capsule to dissolve and how much residue is remaining. As shown in FIGS. 10A to 10D, the polyvinyl alcohol capsules dissolved in about 4 minutes and 30 seconds and the mechanical actuator was able to be deployed from the capsule. There was minimal residue in from the polyvinyl alcohol capsules. In contrast, FIGS. 10E to 10H show that the HPMC capsules take almost twice as long to dissolve (i.e., 8 minutes) and left substantial residue. The HPMC capsule swells and remains on the mechanical actuator, thus affecting deployment. The residue can prevent or interfere with deployment of a payload from the HPMC capsule. For example, the residue may interfere with deployment of a needle delivery system in the intestine. The needles can be buried in the capsule residue and fail to pierce into a sufficient depth in the intestinal tissue (e.g., penetrating into the submucosa layer) for delivery of a therapeutic.

### Moisture Protection Properties of Capsules

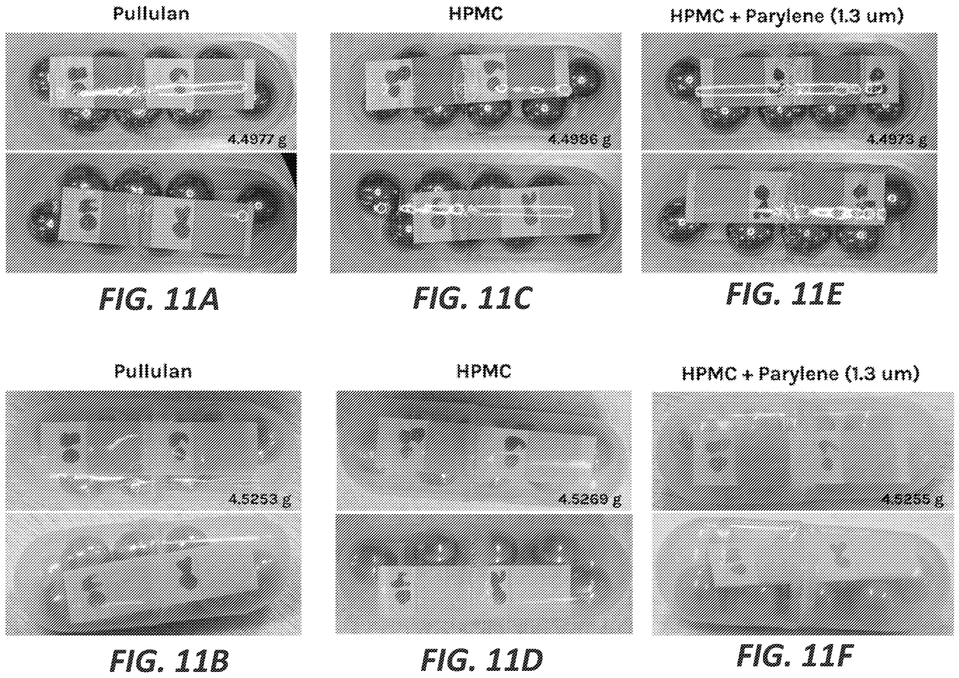

The moisture barrier properties of capsules comprising different materials were tested in soak tests to determine the amount of moisture that enters the capsule. FIGs. 11A and 11B provide capsules made from pullulan, FIGs. 11C and 11D provide capsules made from HPMC, and FIGs. 11E and 11F provide capsules made from HPMC with an inner layer comprising parylene. The inner layer of Test Example 3 comprised a 1.3 micron film of parylene. Test Examples 1-3 each included moisture indicators in the capsules as shown in FIGS. 11A-11F. FIGS. 11A-11F provide images of capsules before soaking comprising Test Example 1 (pullulan; FIG. 11A), Test Example 2 (HPMC; FIG. 11C), and Test Example 3 (HPMC with an inner layer comprising parylene; FIG. 11C). The inner layer of Test Example 3 comprises a 1.3 micron film of parylene. Each of the capsules include an outer layer comprising Eudragit. Each moisture indicator square is marked to its corresponding relative humidity value (20, 40, 60, 80% RH). A square remains blue below its marked relative humidity, and fades to pink at or above its marked relative humidity.

The capsules were soaked in a simulated gastric fluid for 1 hour. The visibility studies are shown in FIGS. 11B, 11D, and 11F. As shown in FIGS. 11B, 11D, and 11F, the inner layer comprising parylene substantially reduced moisture and vapor ingress compared to commercial capsules including pullulan or HPMC alone. In FIG. 11E (before soaking) and FIG. 11F (after soaking), humidity inside the capsule reached 40-60% (60% square is still light blue) after 1 hr gastric soak. In other words, the humidity inside the capsule remains < 60% RH after 1 hr gastric soak when the capsule had a parylene coating as the moisture barrier.

### Capsules including Kollicoat^{®} IR

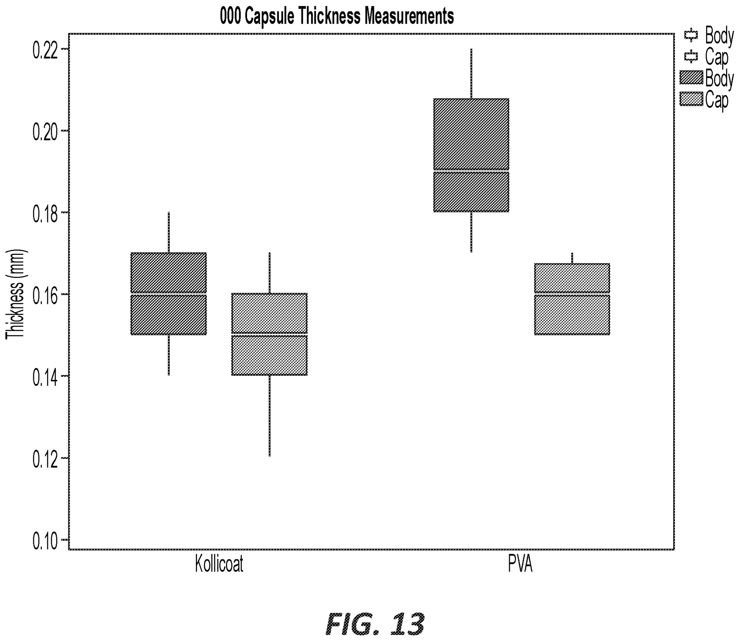

Sample capsules were produced to determine the properties of Kollicoat^{®} IR for producing one or more layers of a capsule. FIG. 13 shows the thickness of Kollicoat^{®} IR capsules compared to PVA capsules. The capsules produced from Kollicoat^{®} IR exhibited more uniform coating thickness than the PVA capsules. Specifically, the Kollicoat^{®} IR had a narrower thickness window than capsules produced from PVA. Kollicoat^{®} IR has a ligher viscosity that PVA which allows for more even coating thickness due to less overflow during the curing process. This produces capsules with precise and controlled coating thickness. For capsules including a layer of Kollicoat^{®} IR, additional coating steps (e.g., dipping into polymer solution) were needed to achieve a similar thickness than PVA capsules. As shown in FIG. 13, the thickness of the body and cap of the capsule produced from Kollicoat^{®} IR was less than the capsules produced from PVA for the same number of coating applications, but exxhibited improved uniformity.

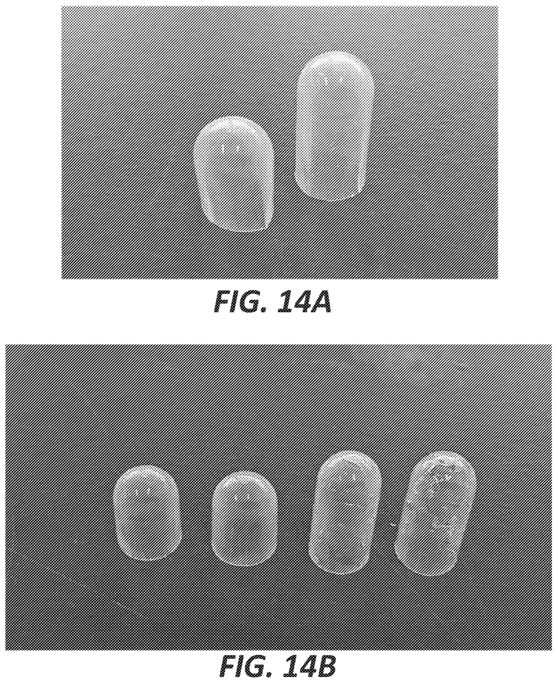

FIG. 14A show images of Kollicoat^{®} IR capsules coated on a parlyene layer and FIG. 14B show images of Kollicoat^{®} IR capsules without a parylene layer. Each of the capsules shown in FIG. 14A and FIG. 14B were produced by dip coating to produce the Kollocoat layer and had a thickness from *0.14-0.*17 mm. Each of the capsules were scored for release. The Kollicoat^{®} IR capsules demonstrated proper wetting and adhesion on micro-90 treated rods. The Kollicoat^{®} IR capsules without parylene (FIG. 14B) demonstrated cracking along the scoring lines and wrinkling. For example, FIG. 14B shows that the Kollicoat^{®} IR capsules produced without a parylene layer exhibited wrinkling. FIG. 14A shows Kollicoat^{®} IR that was coated on a parylene layer did not exhibit any wrinkling. The Kollicoat^{®} IR polymer exhibited good adhesion to the parlyene layer and had good mechanical robustness.

Sample capsules were tested to determine dissolution properties of Kollicoat^{®} IR capsules. Specifically, in-vivo *dissolution* testing in pig intestines was performed to investigate the dissolution rates of capsules including a Kollicoat^{®} IR layer compared to conventional pullulan capsules. Table 2 provides the sample capsule compositions for Examples 4 and 5 and Comparative Examples 6 and 7. Each of the Comparatives Examples 6 and 7 included pullulan as the base capsule material.

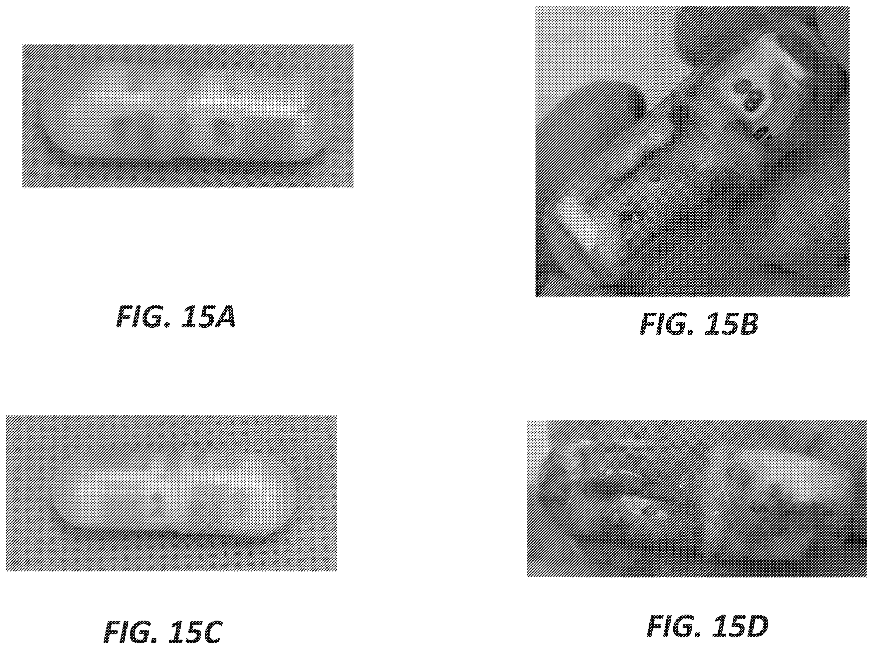

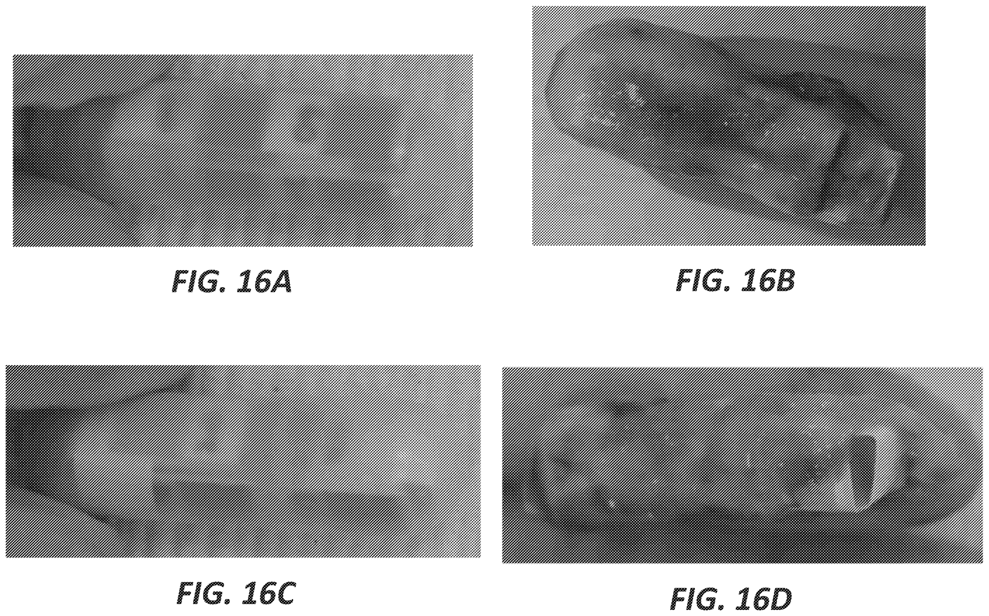

FIGS. 15A-15D and 16A-16D shows images of capsules subjected to in-vivo dissolution tests for capsules including a Kollicoat^{®} IR layer (FIGS. 15A-15D) and a pullulan layer (FIGS. 16A-16D) according to some embodiments of the present disclosure.

| TABLE 2 | | | | |
|---|---|---|---|---|
| | Base Capsule Material | Enteric Coating | Overcoat Layer | Inner Layer |
| Comparative Example 6 | Pullulan | EUDRAGIT^{®} L 100-55 *+ Talc +* DBS | EUDRAGIT^{®} L 100-55 + DBS | - |
| Comparative Example 7 | Pullulan | EUDRAGIT^{®} L 100-55 *+ Talc +* DBS | EUDRAGIT^{®} L 100-55 + DBS | - |
| Example 4 | Kollicoat IR | EUDRAGIT^{®} L 100-55 *+ Talc +* DBS | EUDRAGIT^{®} L 100-55 + DBS | Parylene Layer |
| Example 5 | Kollicoat IR | EUDRAGIT^{®} L 100-55 *+ Talc +* DBS | EUDRAGIT^{®} L 100-55 + DBS | Parylene Layer |

Table 3 provides the dissolution results for Comparative Examples 6 and 7 and Examples 4 and 5. FIGS. 15A and 15C show the capsules of Examples 4 and 5 before soak testing at T₀ and FIGS. 16A and 16C show the capsules of Comparative Examples 6 and 7 before soak testing at T₀. After soak testing for 47 minutes, Examples 4 and 5 had slight enteric removal. After soak testing for 50 minutes, Comparative Examples 6 and 7 *exhibitde* deformation of the capsule. At T2, Examples 4 and 5 and Comparative Examples 6 and 7 had significant deformation. Suprisingly, Examples 6 and 7 demonstrated faster dissolution rates with minimal residue. Specifically, Examples 6 and 7 dissolved much more than Comparative Examples 6 and 7 at T3 (pullulan remaining a rather mechanically intact film that could actually resist some stretching). The capsules of Examples 6 and 7 have improved dissolution properties (e.g., faster) for release of a payload in the gastrointestinal tract than Comparative Examples 6 and 7.

| TABLE 3 | | | | |
|---|---|---|---|---|
| Time | T₀ | T1 = 47 minutes | T2 = 1 hour and 12 minutes | T3 = 1 hour and 28 minutes |
| Example 5 | See FIG. 15A | Slight enteric removal from handling | Deformation on the dome. | See FIG. 15B |
| Example 6 | See FIG. 15C | Slight enteric removal from handling | Deformation on the dome. | See FIG. 15D |
| Comparative Example 6 | See FIG. 16A | Deformation on the dome. | Significant deformation on the dome. | See FIG. 16B |
| Comparative Example 7 | See FIG. 16C | No deformation. | Some deformation on the dome. | See FIG. 16D |

The foregoing description, for purposes of explanation, used specific nomenclature to provide a thorough understanding of the described embodiments. However, it will be apparent to one skilled in the art that the specific details are not required in order to practice the described embodiments. Thus, the foregoing descriptions of specific embodiments are presented for purposes of illustration and description. They are not intended to be exhaustive or to limit the described embodiments to the precise forms disclosed. It will be apparent to one of ordinary skill in the art that many modifications and variations are possible in view of the above teachings.

Aspects of the disclosure are summarised in the following clauses, which form part of the description.
1. A method of producing a capsule, the method comprising: providing a mold; depositing parylene on the mold to form a parylene layer; scoring the parylene layer; and coating the parylene layer with a polymer to produce a capsule.
2. The method of clause 1, further comprising polishing a surface of the mold to ease release of the capsule.
3. The method of clause 1, further comprising applying a release agent on the mold before depositing the parylene layer, and wherein the release agent is Micro90.
4. The method of clause 1, wherein scoring the parylene layer comprises cutting the parylene layer to form a pattern on opposing sides of the capsule.
5. The method of clause 1, further comprising treating an outer surface of the parylene layer with oxygen plasma prior to coating with the polymer.
6. The method of clause 1, wherein a thickness of the parylene layer ranges from 0.5 µm to 30 µm, and wherein a thickness of the polymer layer ranges from 100 µm to 250 µm.
7. The method of clause 1, wherein depositing the parylene layer comprises chemical vapor deposition of parylene on the mold.
8. The method of clause 1, wherein depositing the parylene layer comprises: depositing, in a first deposition step, parylene on the mold to produce a primer layer; treating the primer layer with a release agent; and depositing, in a second deposition step, parylene on the primer layer to produce the parylene layer.
9. The method of clause 1, wherein the polymer is polyvinyl alcohol.
10. The method of clause 1, further comprising applying an enteric coating on the polymer layer, wherein the enteric coating comprises anionic methacrylate copolymer, talc, and plasticizer, and wherein a weight ratio of talc to anionic methacrylate copolymer ranges from 0.5:1 to 10:1.
11. The method of clause 1, further comprising encapsulating a needle delivery system in the capsule.
12. A capsule comprising: a first layer comprising parylene; a second layer adjacent the first layer, the second layer comprising a polymer; and a third layer adjacent the second layer, the third layer comprising an enteric coating.
13. The capsule of clause 12, wherein the first layer is scored to form a bi-fold pattern on opposing sides of the capsule.
14. The capsule of clause 12, wherein a thickness of the parylene layer ranges from 0.5 µm to 30 µm.
15. The capsule of clause 12, wherein the second layer comprises a plurality of layers, wherein at least one of the plurality of layers comprises polyvinyl alcohol, and wherein the second layer has a thickness from 100 µm to 250 µm.
16. The capsule of clause 12, wherein the polymer comprises one or more of hydroxypropyl methylcellulose, pullulan, polyvinyl alcohol, or polyvinylpyrrolidone.
17. The capsule of clause 12, wherein the enteric coating comprises anionic methacrylate copolymer, talc, and plasticizer, wherein talc comprises 40 wt. % to 80 wt. % of the enteric coating, based on the total weight of the enteric coating, and wherein the anionic methacrylate copolymer comprises 20 wt. % to 60 wt. % of the enteric coating, based on the total weight of the enteric coating.
18. The capsule of clause 12, wherein the capsule does not comprise pullulan or hydroxypropyl methylcellulose.
19. The capsule of clause 12, further comprising a fourth layer comprising anionic methacrylate copolymer and plasticizer.
20. The capsule of clause 12, wherein: the polymer of the second layer comprises polyvinyl alcohol; the enteric coating comprises anionic methacrylate copolymer, talc, and plasticizer; and a fourth layer comprises anionic methacrylate copolymer and a plasticizer.

## Claims

1. A method of producing a coating composition, the method comprising:
providing a solvent comprising a plasticizer;
adding anionic methacrylate copolymer to the solvent;
adding talc to the solvent; and
mixing the anionic methacrylate copolymer and talc in the solvent to produce the coating composition.

2. The method of claim 1, wherein the plasticizer is hydrophobic.

3. The method of claim 1 or 2, wherein the plasticizer comprises dibutyl sebacate.

4. The method of any preceding claim, wherein the solvent comprises one or more of acetone, isopropyl alcohol, and water.

5. The method of any preceding claim, wherein the plasticizer is present in an amount from 5 wt. % to 25 wt. %, based on the weight of the anionic methacrylate copolymer.

6. The method of any preceding claim, wherein a weight ratio of talc to anionic methacrylate copolymer ranges from 0.5:1 to 10:1.

7. The method of any preceding claim, wherein a weight ratio of talc to anionic methacrylate copolymer ranges from 1:1 to 3:1.

8. The method of any preceding claim further comprising mixing the anionic methacrylate copolymer and talc together prior to adding them to the solvent.

9. The method of any preceding claim further comprising mixing the anionic methacrylate copolymer and talc in a first solvent and then adding to the solvent comprising the plasticizer, wherein the first solvent comprises water, acetone, and/or isopropyl alcohol.

10. An enteric coating composition comprising:
an anionic methacrylate copolymer,
talc, and
plasticizer,
wherein talc comprises 40 wt. % to 80 wt. % of the enteric coating, based on the total weight of the enteric coating, and
wherein the anionic methacrylate copolymer comprises 20 wt. % to 60 wt. % of the enteric coating, based on the total weight of the enteric coating.

11. The enteric coating of claim 10, wherein a weight ratio of talc to anionic methacrylate copolymer ranges from 0.5:1 to 10:1.

12. The enteric coating of claim 10 or 11, wherein the plasticizer is present in an amount from 5 wt. % to 25 wt. %, based on the total weight of the anionic methacrylate copolymer.

13. The enteric coating of any one of claims 10 to 12, wherein the plasticizer is hydrophobic.

14. The enteric coating of any one of claims 10 to 13, wherein the plasticizer comprises dibutyl sebacate.

15. A capsule comprising the enteric coating of any one of claims 10 to 14.
